# EUROPEAN PATENT APPLICATION

(11) **EP 1 901 196 A2**
(43) Date of publication of application: **19.03.2008**
(21) Application number: 07017806.6
(22) Date of filing: 12.09.2007
(51) Int. Cl.: G06F 21/24

(54) **Method of and system for security and privacy protection in medical forms**

(30) Priority: 12.09.2006 US 825329 P; 12.09.2006 CA 2559523
(71) Applicant: Trialstat Corporation, Ottawa ON K2C 3W7 (CA)
(72) Inventor: El Kadri, Nour A., Gatineau, Quebec J9A 3B3 (CA); Hein, Richard Anthony, Kanata, Ontario K2L 2A4 (CA); El Emam, Khaled M., Ottawa, Ontario (CA); Neri, Emilio Giuseppe, Orleans, Ontario K1C 1X8 (CA); Alkarkhi, Mazin, Gatineau, Quebec J9A 3C1 (CA); Tsarouchas, Akaterina, Ottawa, Ontario K1S 1X9 (CA)
(74) Representative: Schaad, Balass, Menzl & Partner AG

(57) **Abstract**

The present invention relates to electronic medical forms and, in particular, to a method of and system for security and privacy protection in medical forms. The invention provides a medical form system and method that allows clients dealing with highly sensitive data to ensure that particular data entered is never accessible to certain other parties (or all other parties). In the preferred embodiment, this is done by encrypting and decrypting certain data only on the client and preventing decryption of the data on the server-side. Other systems do provide security in the form of SSL connections from the client to the server and enforce good security practices, but they do not provide client-side encryption.

## Description

### FIELD OF INVENTION

The present invention relates to electronic medical forms and, in particular, to a method of and system for security and privacy protection in medical forms.

### BACKGROUND OF THE INVENTION

Forms, whether paper or electronic, are commonly used in the collection of data. The generation and management of forms used in medical activities, such as pharmaceutical trials, is known for being particularly costly and consuming considerable human resources due to their unique requirements.

Medical forms are typically completed by subject and/or their caregivers, and circulated through a number of different parties, such as data entry clerks, data analysis consultants, Internet Service Providers (ISPs), hospitals, pharmaceutical companies, government regulators and the like. Medical forms often contain highly sensitive data requiring that certain data never be accessible to certain other parties, and possibly all parties other than one who entered the sensitive data. There is currently no system available that provides the functionality to protect such sensitive data. There are systems which provide security in the form of SSL connections from the client to the server, and enforce good security practices, but these systems do not distinguish between the different data.

There is therefore a need for an improved method of and system for security protection in medical forms.

### SUMMARY OF THE INVENTION

It is an object of the invention to provide a method of and system for security and privacy protection in medical forms, which obviates or mitigates at least one of the disadvantages described above.

The invention provides a medical form system and method that allows clients dealing with highly sensitive data to ensure that particular data entered is never accessible to certain other parties (or all other parties). In the preferred embodiment, this is done by encrypting and decrypting certain data only on the client and preventing decryption of the data on the server-side.

As noted in the Background, other systems do provide security in the form of SSL connections from the client to the server and enforce good security practices, but they do not provide client-side encryption.

This document discusses the requirements and the high-level software design specifications necessary to provide client-side encryption operational in a medical form system such as the ClinicalAnalytics (CA) software version 3.0 available from TrialStat Corporation. The feature set of this system includes: User Management, Key Management, Form/Question Designer, Renderer and Database Requirements.

This summary of the invention does not necessarily describe all features of the invention.

### BRIEF DESCRIPTION OF THE DRAWINGS

These and other features of the invention will become more apparent from the following description in which reference is made to the appended drawings wherein:
Figure 1 presents a flow chart of a method of client-side encryption key management, in accordance with an embodiment of the present invention;
Figure 2 presents a flow chart of a method of client-side encryption data flow, in accordance with an embodiment of the present invention;
Figure 3 presents a screen shot of an exemplary site/user management interface, in accordance with an embodiment of the present invention;
Figures 4-7 present screen shots of exemplary key management interfaces, in accordance with an embodiment of the present invention; and
Figure 8 presents a screen shot of an exemplary renderer user interface, in accordance with an embodiment of the present invention.

### DETAILED DESCRIPTION

The present invention will be further illustrated by means of the following examples.

This exemplary implementation requires client-side ActiveX controls which is supported by Internet Explorer 5.5+ browsers and above, but is typically not supported in other browsers. The following are not explicitly described but are easily accommodated in view of the teachings herein:
· Mobile devices support (requires writing a POD or other method)
· Browsers other than IE 5.5+
· Allowing encryption to be enabled/disabled on an existing project that already has data (we will add this to the Records Management feature-set for future releases)
· Changing the Master Key
· More than one supported encryption administrator per center.

### Site, User and Encryption Key Management Requirements

General Description

Key Management concerns how the administrators and users deal with the creation and distribution of encryption keys and passphrases.

Project/Center Management concerns the steps necessary to configure center, project and user permissions and encryption support. Project Administrators are responsible for configuring encryption support across a Project/Center combination, which includes projects and centers and users within those centers.

Requirements

9.2.1 Key Management Requirements:

9.2.1.1 Clients require an interface to be able to manage encryption settings.

9.2.1.2 Project Administrators must be able to select Centers and Users that will support and use encryption respectively.

9.2.1.5 A Master Key will be randomly generated that is used to encrypt and decrypt data within a study.

9.2.1.6 The Master Key is the only key that ultimately may encrypt and decrypt data within a center correctly.

9.2.1.7 There will be one Master Key per Center.

9.2.1.8 The Project Administrator is responsible for storing the Master Key for each Center, either in a secure location or in escrow. The details of preserving this passphrase must be dealt with by good practices and SOPs. If the Project Administrator loses the Master Key, the data is unrecoverable.

9.2.1.9 Each user assigned to a center that will use encryption will be assigned a User Key.

9.2.1.10 Each user must use their own system login as a passphrase for their User Key.

9.2.1.11 The User Key is unique to each user.

9.2.1.12 Only a Project Administrator must be able to enable encryption settings on a Center within the Project he/she administers,

9.2.1.13 Only the Project Administrator who originally generates a Master Key for the Center will be allowed to manage User Keys for encryption within that Center. That is, there is one encryption administrator per Center, on a first-come, first-serve basis.

9.2.1.14 A Master Key can only be generated once per Center. To reset a Master Key, the client must contact Technical Support.

9.2.1.15 The Center Key Management UI (user interface) will be accessible only to Project Administrators.

9.2.1.16 User key management UI will be accessible only by the original Project Administrator that initially generated the Master Key.

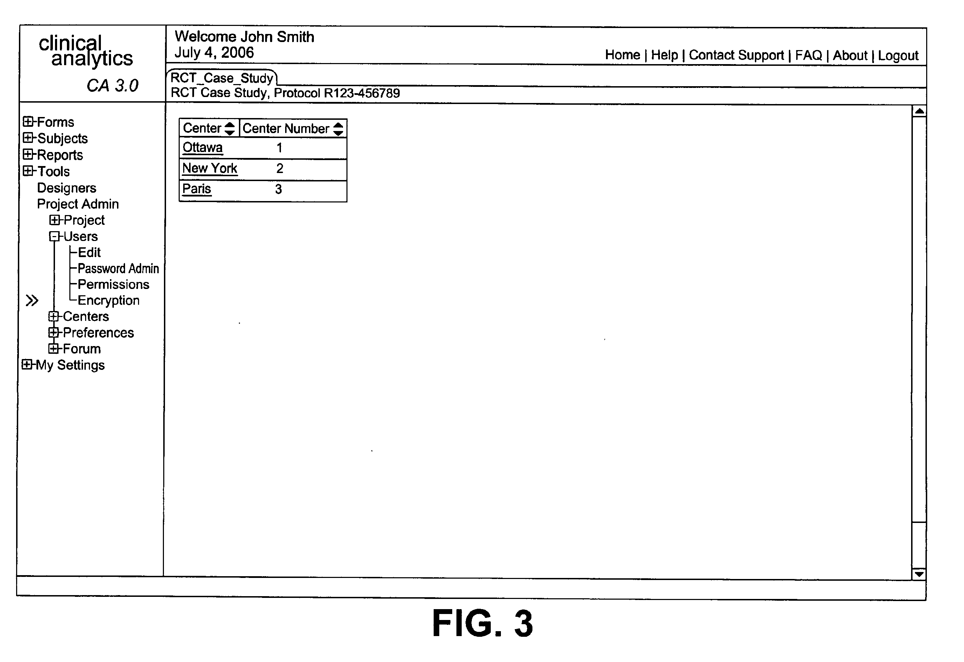

9.2.1.17 An Encryption menu item must be included in the Navigator under Project Admin - Centers - Encryption. See Figure 3 for an exemplary user interface depicting this.

9.2.1.18 Project Administrator(s) must be able to select the Centers to which they will enable encryption support within their Project. See Figure 3.

9.2.1.18.1.1 Only display one Center at a time.

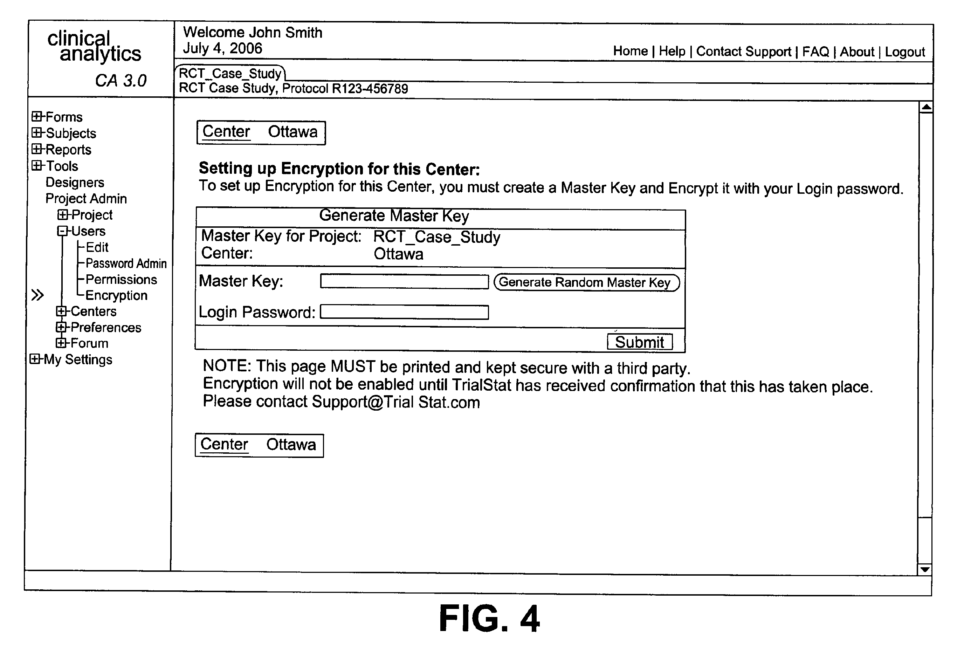

9.2.1.19 Project Administrator(s) must be able to create the Master Key(s) for the Center. See Figure 4 for an exemplary user interface depicting this.

9.2.1.20 Master Keys should be randomly generated. A button is provided on the page in Figure 4 to generate a random key.

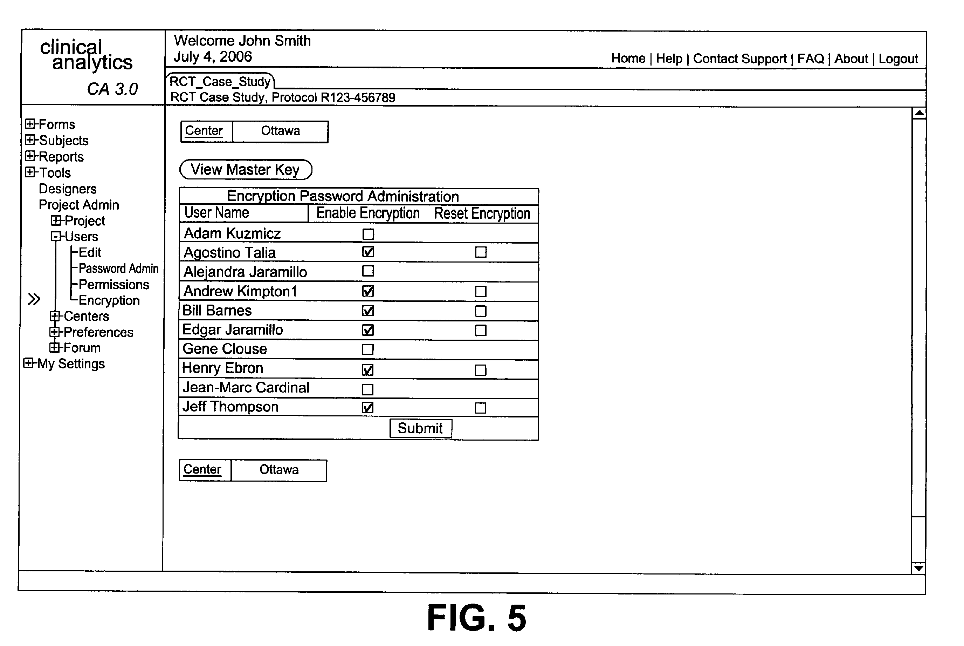

9.2.1.21 The Project Administrator who generated the Master Key will select the users who should be allowed access to encrypted data. See Figure 5.

9.2.1.22 The system auto-generates a pair of passwords/keys that are used to verify the user's rights when they first try to access the Master Key. These are called the Email Key and Verbal Key. One part is stored in the system and is accessible by the Project Administrator. The Other part is emailed to the user.

9.2.1.22.1 Only the verbal key is stored in the system database for security reasons. The email key must not be stored in the database as rejoining both keys allows access to the decrypted Master Key.

9.2.1.23 Users who have had encryption enabled will be prompted to enter both the Email Key and the Verbal Key, as well as their system login password when they attempt to access an encrypted form for the first time - this is when a User Key is created.

9.2.1.24 The Project Administrator provides the Verbal Key to the user on request, in person or on the telephone after confirming the identity of the user,

9.2.1.25 User only has to login to the system after initially setting up encryption to access encrypted forms.

9.2.1.26 The system keeps a Session Key encrypted in a cookie to track the users encryption rights, as well as use that Session Key to decrypt data in the forms.

9.2.1.27 The master key is never visible unencrypted on the server.

9.2.1.28 Data in encrypted fields are never visible unencrypted on the server, including any reports, exports, etc... that may be executed on the server.

### Center Encryption Management Use Case

Actors: Project Administrator

Preconditions; Project has encryption enabled manually in the Projects table Encryption flag, by Professional Services or a DBA.

9.2.2 The Project Administrator enables encryption by navigating to Project Admin - Centers - Encryption.

9.2.2.1 The System displays the Project Admin - Centers - Encryption Menu item.

9.2.2,2 The System displays the Project Admin - Centers - Encryption menu item.

9.2.2.3 The user navigates to the Project Admin - Centers - Encryption menu item.

9.2.2.4 The System displays the Centers belonging to the selected Project. See Figure 3.

9.2.2.5 The User selects a center.

9.2.2.6 System displays the Center and sets up encryption for that center.

9.2.2.7 If a Center does not have Encryption enabled:

9.2.2,7,1 System displays the Master Key field along with a field to enter the Project Administrator's system password.

9.2.2.7.2 The user creates the Master Key for the Center, by clicking "Generate Random Master Key". They must record the master key as there is no way to recover it.

9.2.2.7.3 The System auto-generates a random key for Triple DES2 encryption. This is referred to as the "MASTER KEY", distinguishing it from the set of user passphrases or user keys, which includes the administrator's passphrase/user key as well.

9.2.2.7.4 User enters their system password.

9.2.2.7.5 User clicks "Submit" to apply the changes.

9.2.2.7.6 System validates data: Master Key and the user's system password must be provided. The System compares the Project Admin's system password hash to validate:

9.2.2.7.7 System provides error message if the password is incorrect.

9.2.2.7.8 If validated, the System stores the system password, encrypted by the SessionId, in a cookie and then encrypts the new Master Key with the user's system password - this creates the first User Key.

9.2.2.7.9 The System warns the project administrator that this change is permanent, and allows them to cancel.

9,2.2.7.10 System stores the User Key in the UserCenters table and flags the user as Admin, setting the Admin column to "Y".

9.2.2.8 If a Center has previously had Encryption enabled, the Master Key must not be changed. Changes are not allowed, and the screen should state this clearly.

### Encryption Support Login Use Case

Actors: System User, Professional Services

Preconditions: Center Encryption Management Use Case is complete. There is a Generated Master Key for the Center. The Encryption status flag for the center administrator is set to "P" (pending).

9.2.3 The User logs into the system and the system checks if they require encryption support.

9.2.3.1 System checks if the user is a Subject:

9.2.3.2 If the user is a Subject:

9.2.3.2,1 They cannot use encryption support in this release, so the system logs the Subject in as usual with no encryption support.

9.2.3.3 If the user is not a Subject the System checks the user's login id and password as usual, and determines if the user has a Encryption flag in the UserCenters table that matches their user id.

9.2.3.4 If the user requires encryption support (If the Encryption flag is "P" or "Y"), the system renders the ASPEncrypt <object> into the login page.

9.2.3.4.1 The browser prompts the user to install the ASPEncrypt ActiveX control.

9.2.3.4.2 User chooses to install the ActiveX control.

9.2.3.4.3 If the user does not install the ActiveX control, the features will not work and they are treated as a user without encryption support. Continue with 9.2.3.6.

9.2.3.4.4 System captures the user's login password during login and encrypts it with the SessionId, creating the user's passphrase for their User Key, and stores the encrypted system password in a cookie.

9.2.3.5 If the user does not require encryption support, the user is treated as usual without encryption support. Encrypted data will appear garbled or say "This data is encrypted".

9.2.3.6 System continues to login the user as normal.

### User Encryption Management Use Case

Actors: Project Administrator

Preconditions: Center has a Master Key generated and stored securely in escrow.

9.2.4 Project Administrator logs into the system to set which users may use encryption.

9.2.4.1 System captures the user's login password during login and encrypts it with the SessionId, creating the user's passphrase for their User Key, and stores the encrypted system password in a cookie.

9.2.4.2 Project Admin goes to Project Admin-Users-Encryption menu item and selects a Center.

9.2.4.3 System checks if the Project Admin is an Admin for encryption, by checking the UserCenters Admin column for a value of"Y". If it is there, they are the person who generated the Master Key.

9.2.4.4 If the Project Admin is NOT the same admin who generated the Master Key for the Center:

9.2.4.5 The System displays a message saying, "Only the administrator who created the Master Key for the selected Center may administer encryption support for users."

9.2.4.6 If the Project Admin is the same admin that generated the Master Key for the Center.

9.2.4.7 The Project Administrator will select the users who should be allowed access to encrypted data. See Figure 5 for an exemplary user interface to achieve this. They can either select Set or Reset - the workflow is identical it just shows who already has it.

9.2.4.8 The System must auto-generate a pair of passwords/keys, called Key1 - Email Key and Key 2 - Verbal Key for each User selected.

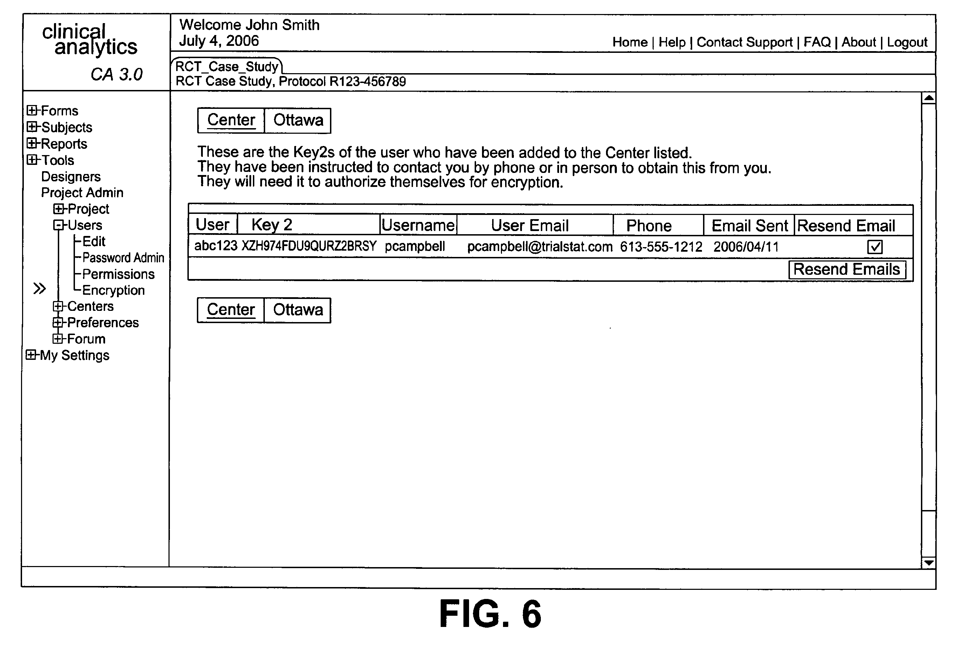

9.2.4.9 The System redirects the Project Administrator to a page used to display the Verbal Keys that were created for the user. See Figure 6 for an exemplary user interface. This page will list all of the Users that were just selected and their corresponding Verbal Keys, which must unique.

9.2.4.10 The System uses the current user's (the Project Admin) Session Key from the cookie to decrypt the Master Key (from the Admin's MasterKey column in the database), then REENCRYPTS the MasterKey using the generated Email and Verbal key combined to make a passphrase. This acts as a temporary password to access the Master Key.

9.2.4.11 The System will send the users emails with the Key1 - Email Key half of the password and display a success notification to the Project Administrator that the changes were successful and explain the usage of the second half of the key (Key 2 - Verbal Key). The System stores the VerbalKey (Key2) in the database (UserCenters table, Key2 column).

### Workflow Diagram

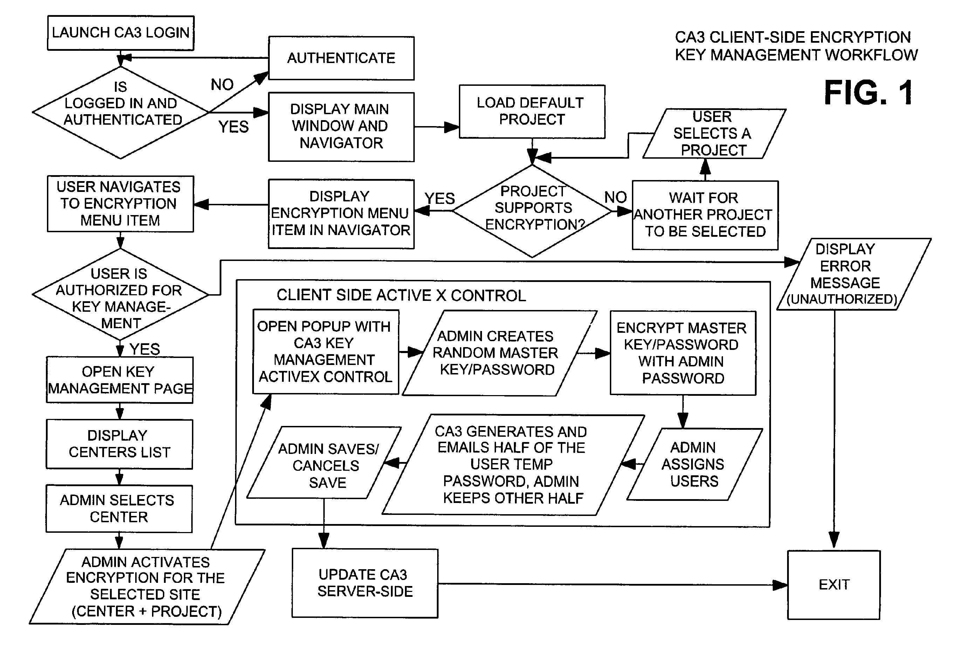

The flowchart of Figure 1 depicts the key management workflow for the client-side encryption feature of the system. The shaded blocks represent client-side activities.

### Form/Question Designer Requirements

Forms and Questions must be configured to support encryption. At the database level this requires some changes, but basically just sets a flag. The flags will be set manually in the database for specific center/project/form/questions that require encryption support in this release. Optionally, one could allow the user to enable/disable encryption for any project/form/question they wish.

1. There are no Form/Question Designer Requirements. We do not allow the user to change encryption settings on a form in the initial release. We manually configure Project/Question encryption settings for the client(s) that require it for this release.

### Renderer Requirements

The Renderer must be able to identify users that are allowed to encrypt/decrypt, and gracefully display the fact that a user cannot access encrypted fields when such an event occurs. It must also perform all encryption/decryption on the client-side via a 3rd-party ActiveX control, ASPEncrypt.

Requirements

9.2.5 Renderer Requirements:

9.2.5.1 Only supported in IE 5.5+. Palm and other mobile devices are not currently supported but can be accommodated.

9.2.5.2 The Renderer encrypts and decrypts data automatically for users that have encryption enabled and configured correctly.

9.2.5.3 Identify forms that support encryption.

9,2,5.4 Authenticate and check the Authorization of the user to discover if they can encrypt/decrypt data.

9.2.5.5 Challenge the user with a login prompt if they have not entered their passphrase for the session. Obsolete since it is renewed with each login session.

9.2.5.6 Check an encrypted value stored in a cookie for a saved local passphrase, to avoid forcing the user to provide credentials every time they access an encrypted Form. This is the Session Key.

9.2.5.7 Grab all the questions in a given form that are encrypted, display and (re-)encrypt the responses for those questions only.

9.2.5.8 Display a friendly ("Encrypted Data") message inside text fields that are encrypted for unauthorized users. Highlight the fields of encrypted data. For types that do not allow text messages, just highlight the field.

9,2.5.9 Support short and long text, numeric and date/time question types only.

9.2.5.10 Users who are not allowed access to encrypted data must be able to access forms nevertheless.

9.2.5.10.1 Display ****s over the encrypted data when a user is not allowed to decrypt it.

### User Logs in with Encryption Enabled the First Time, OR the User has Changed their system Password Use Case

Actors: Regular User

Prerequisites: User must be a system user with permissions to view the requested Form and access the Project and Center as usual.

9.2.6 The User logs into the system.

9.2.6.1 The System checks the browser settings to make sure ActiveX controls are enabled, and all required settings are OK. If they are not, the System redirects the user to an error page saying "ActiveX controls must be enabled in the Browser. Please contact your system administrator."

9.2.6.2 The System loads the ASPEncrypt ActiveX control.

9,2,6.3 The User must accept the installation of the ActiveX control the first time, and/or every time the browser's downloaded object cache is cleared.

9.2.6.4 The System temporarily stores the User's system login password, encrypted with the SessionId, in a encrypted value in a cookie.

9.2.6.5 The System checks if the User has a PwdChangeFlag set to 'Y' in the CAUSERS table, for ANY Site, then for EACH site performs the following (alternatively, we do this for all the Sites the User is allowed encryption access to at once, using one login, since it's always the system login):

9.2.6.6 If the User changes their encryption passphrase:

9.2.6.6.1 The System displays a screen similar to the Password Reset dialogs for system password changes, with fields for Key1 and Key1 of their passphrase.

9.2.6.6.2 The User must enter in the first half of the passphrase, acquired from an email that was sent to them, into field Key1.

9.2.6.6.3 The User must enter in the second half of the passphrase, acquired over the telephone or in person, from the Project Administrator.

9.2.6.6.4 The System uses the combined Key1 and Key2 fields to decrypt the Master Key(s).

9.2.6.6.5 The System decrypts the User's login from UserData, using the SessionId as the key, and then re-encrypts the Master Key(s) with their system login. This is all transparent to the user.

9.2.6.6.6 The encrypted MasterKey(s) is/are updated for the UserId, ProjectIds and CenterIds of the user - in the CAUSERS table.

9.2.6.6.7 The System redirects the user to their normal start page.

### User Accesses Encrypted Form

Actors: Regular User

Prerequisites: The user must have permission to access the project / center / form as usual. The user has gone through the "User Logs in with Encryption Enabled the First Time, OR the User has Changed their system Password Use Case"

9.2.7 User logs in.

9.2.7.1 The System checks the browser settings to make sure ActiveX controls are enabled, and all required settings are OK. If they are not, the System redirects the user to an error page saying "ActiveX controls must be enabled in the Browser. Please contact your system administrator."

9.2.7.2 The System loads the ASPEncrypt ActiveX control.

9.2.7.3 The User must accept the installation of the ActiveX control the first time, and/or every time the browser's downloaded object cache is cleared.

9.2.7.4 System intercepts the system login password, and encrypts it using ASPEncrypt, using the SessionId as the key. The encrypted password is stored in a cookie.

9.2.7.5 The System redirects the user to their start page.

9.2.7.6 User selects a Form which has encrypted questions.

9.2.7.7 The System checks the current UserId against the list of known users who have access to encrypted portions of the Form. (CAUSERS table.) If the user has access to encrypted data, all question responses are visible. If they do not, we display "Encrypted Data" in text fields, and highlight the non-text fields.

9.2.7.8 The System keeps a list of the encrypted question ids, and answer ids from that form.

9.2.7.9 The System checks the cookie for the encrypted User passphrase.

9.2.7.10 If it is there, it decrypts it using the SessionId as the key.

9.2.7.11 If it is not there, the user must be redirected to the Login page, or sent to an error page.

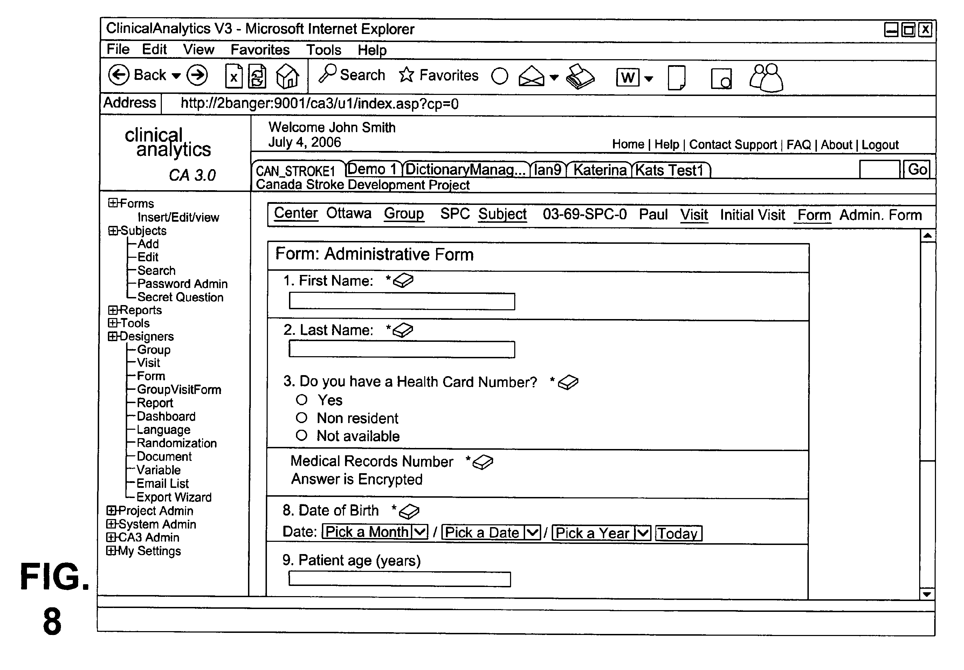

9.2.7.12 The System displays the Form and Questions as in Figure 8, with encrypted questions highlighted.

9.2.7.13 If there is any existing data (like editing a form, rather than creating a new instance), the System uses the decrypted User passphrase to decrypt the Master Key for that particular Site (Center/Project combination). The System then decrypts the data with the Master Key.

9.2.7.14 The User fills in the Form data.

9.2.7.15 The User clicks Submit.

9.2.7.16 The System uses the decrypted User passphrase to decrypt the Master Key for that particular Site (Center/Project combiunation).

9.2.7.17 The System uses the Master Key to encrypt the data in the responses for the matching questions and answer ids in the list referred to in step 4.

9.2.7.18 The System submits the data to the server.

### Database Requirements

Changes to the CliuitcalAnalytics (CA) 3.0 database are required to support encryption. Here is a list of the required changes. Other databases could be modified in a similar manner to facilitate the invention.

Requirements

9.2.8 Database Requirements:

9,2,8,1 The Forms table requires an "Encrypted" boolean flag. `Y' or 'N'.

9.2.8.2 The Projects table requires a "SupportsEncryption" flag. 'Y' or 'N'.

9.2.8.3 The Centers table requires a "SupportsEncryption" flag. 'Y' or 'N'.

9.2.8.4 The CAUsersProjects table requires the following fields:

9.2.8.4.1 MasterKey (formerly "Key") - Image. type (it will be encrypted).

9.2.8.4.2 CenterNo (formerly Site). Foreign Key to Centers table.

9.2.8.4.3 PwdFlag (formerly MustChangePwd). Allow the following flags; 'E' = Enabled, 'D' = Disabled ,'R'=Reset (user has to change password).

9.2.8.4.4 IsEncrypted. - Flag allowing 'Y'=Yes, 'N'=No or 'P'=Pending

9.2.8.5 The Users table requires an "Encryption" flag. 'Y' or 'N'.

9.2.8.6 The Questions table requires an "Encrypted" flag. 'Y' or `N'.

9.2.8.7 The ResponseLink table requires an "Encrypted" flag. 'Y' or `N'.

### API Functions

The system will be provided with the following API functions, but this is not a complete list. The functions are generally self-descriptive:

```
 function EncryptForm() // Encrypts all questions marked for encryption on a form; must
 be called from Renderer//W
 function DecryptForm() // Decrypts all encrypted questions marked for encryption on a
 form//
 function DecryptElements() // For each of the encrypted question fields on the form ...
 function EncryptElements() // For each of the encrypted question fields on the form ...
 function Encrypt(plainText)
 function GetUserKey()
 function Decrypt(cipherText)
 function SetEncryptedBackground(elementName) // Set the background colour of an
 encrypted item.//
 function SetDecryptedBackground(elementName)
 function GenerateRandomMasterKey()
 function EncryptMasterKey(masterKey, ca3Password)
 function EncryptUserKey(userKey)
 function DecryptUserKey() // Decrypts the user key stored in UserData, if it exists. If it
 doesn't it makes one up for the current session. //
 function GetSessionId()
 function LoadSessionKey(userId)
 function SaveSessionKey(userId)
 function CreateCryptoContext()
```

### Data Flow

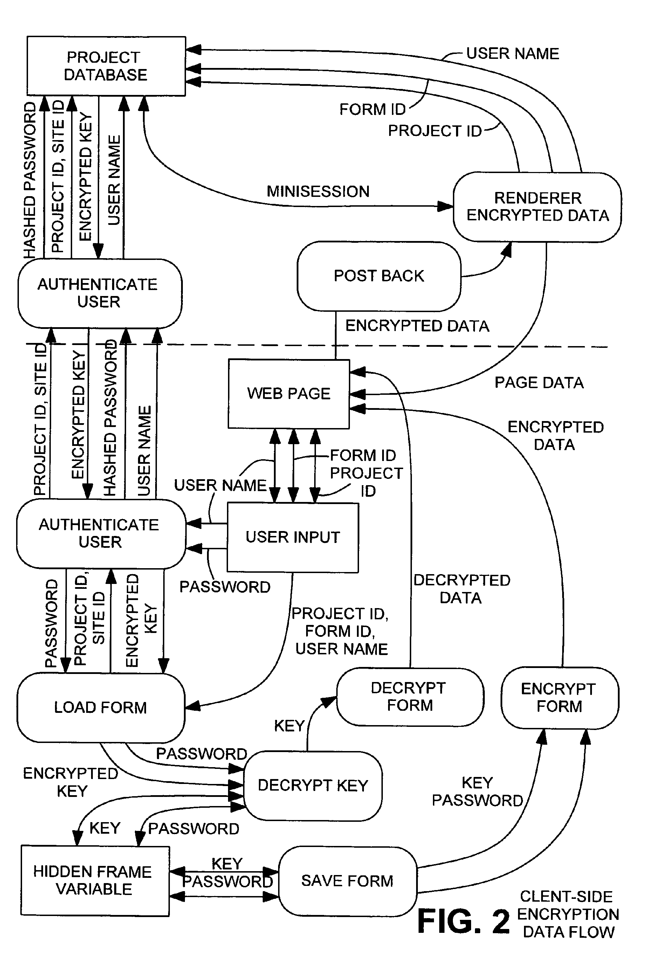

Figure 2 presents a flow chart of the data flow for the encrypted/decrypted data, and is intended to serve as a generalization for any pages that use the client-side encryption features provided by ASPEncrypt. This provides developers with a way to follow the required data elements as they flow from server to client and vice-versa.

The hashed line represents the boundaries between server and client side, the top half is the server-side, the bottom half is the client-side.

If you look at "User Input" in the diagram, you can follow the required data flow from any page (be it the Renderer or key management pages) through the client-side, then to the server, or from the server to the client.

### User Interfaces

Exemplary Site/User Management, Key Management and Renderer user interfaces are presented in Figures 3 through 8.

Figure 3 presents a user interface for Site/User Encryption Management. The Project Administrator must select the Center for which he/she intends to configure encryption support.

Figure 4 - The first time to the Centre, they will be asked to create a Master Key. Then click Generate Master Key, or enter their own Master Key. Enter Login password to authenticate Project Admin and to Encrypt the Master Key. This is the page that should be printed and to go to the Trusted site.

Figure 5 - After setting the Master Keys for a Center and submitting the changes, the Project Administrator is redirected to this page, which displays a list of the users in the previously selected Center.

Figure 6 - the system will auto-generate a pair of passwords that combined are used to decrypt the Master Key. Each user will be sent one half of their passphrase. They must contact the Project Administrator by phone or in person to obtain the second half. This is the same process used to reset a user's password, but it will not reset the system password. If a new user was added in the enable list above or the Reset for a user was selected, an email is sent to the user with Key1, and a Key2 entry is added to the list below. Otherwise, the list below contains the outstanding Key2 pairs, If all the Key1, Key 2 pairs have been entered by users, this list is empty. The time of the last email with Key1 is listed. It can be resent by selecting the check box, and hitting Resend Emails.

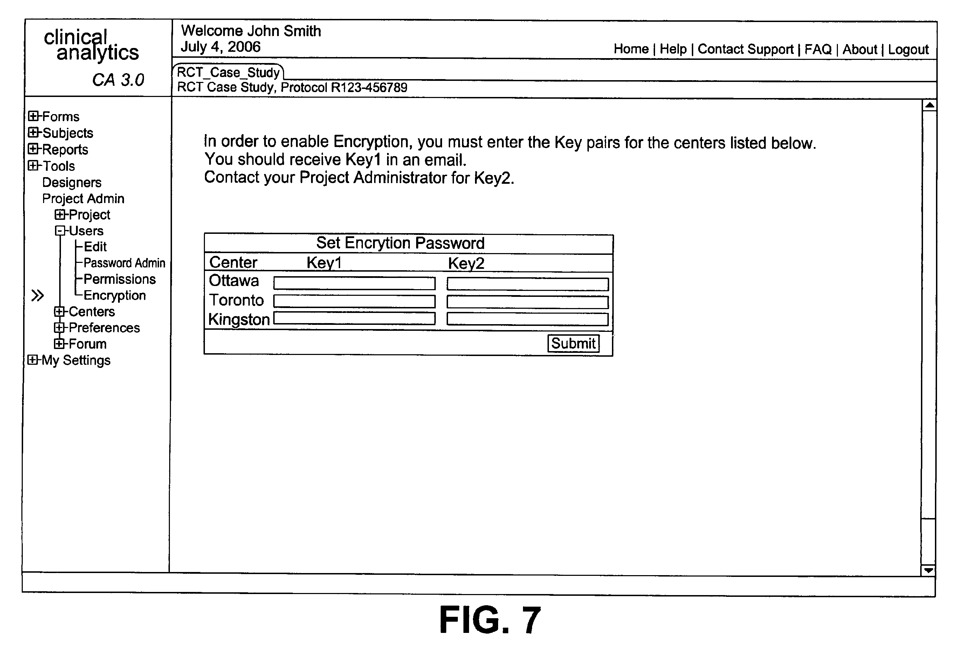

Figure 7 - When a regular user logs on, if they have outstanding Key1/Key2 requests, this screen is presented for the user to enter their key pairs.

Figure 8 - The Renderer will display encrypted questions with special highlighting. If the user has permission to view encrypted data, they will see the encrypted fields, if they do not, the text will be displayed as "Encrypted". For non-text answers, we will display another highlight colour with a pop-up tooltip that says "Encrypted Data".

### Conclusions

The present invention has been described with regard to one or more embodiments. However, it will be apparent to persons skilled in the art that many alternatives, modifications, and variations can be made without departing from the scope of the invention as defined in the claims.

The method steps described may be embodied in sets of executable machine code stored in a variety of formats such as object code or source code. Such code is described generically herein as programming code, or a computer program for simplification. Clearly, the executable machine code may be integrated with the code of other programs, implemented as subroutines, by external program calls or by other techniques as known in the art.

Embodiments of the invention may be executed by a computer processor or similar device programmed in the manner of method steps, or may be executed by an electronic system which is provided with means for executing these steps. Similarly, an electronic memory medium such computer diskettes, CD Roms, Random Access Memory (RAM), Read Only Memory (ROM) or similar computer software storage media known in the art, may be programmed with code to execute such method steps. As well, electronic signals representing these method steps may also be transmitted via a communication network.

All citations are hereby incorporated by reference.

## Claims

1. A method of medical trial form management comprising the steps of:
generating an electronic medical trial form including fields which may be electronically populated by a user;
populating said fields with data values; and
encrypting data values in predetermined ones of said fields.

2. The method of claim 1 wherein said step of populating is performed by a user, via a graphic user interface.

3. The method of claim 1 wherein said predetermined ones of said fields are fields which comprise personal information, whereby all parties will be able to access unprotected data value, but only authorized individuals will be able to access said encrypted data value.

4. A system comprising:
a user computer;
a remote server;
a remote database; and
a communication network for transferring data between said user computer and said remote server, and between said remote server and said remote database;
said user computer being operable to:
generate an electronic medical trial form including fields which may be electronically populated by a user;
populate said fields with data values; and
encrypt data values in predetermined ones of said fields.
